# EUROPEAN PATENT APPLICATION

(11) **EP 4 465 305 A1**
(43) Date of publication of application: **20.11.2024**
(21) Application number: 23173430.2
(22) Date of filing: 15.05.2023
(51) Int. Cl.: G16H 50/20

(54) **METHODS AND SYSTEMS FOR USING AND TRAINING CLINICAL SUPPORT ALGORITHMS**

(71) Applicant: Koninklijke Philips N.V., 5656 AG Eindhoven (NL)
(72) Inventor: NEUMANN, Rolf, 5656 AG Eindhoven (NL)
(74) Representative: Philips Intellectual Property & Standards

(57) **Abstract**

A method for collecting data indicative of the health of a subject. The method comprises obtaining raw physiological data of a subject and inputting the raw physiological data into a clinical support algorithm, where the clinical support algorithm is trained on prior raw physiological data of one or more subjects. A first data stream containing the output of the clinical support algorithm is generated. The raw physiological data is also separately modified using caregiver settings to generate a second data stream containing the modified physiological data.

## Description

### FIELD OF THE INVENTION

The invention relates to clinical support algorithms. In particular, the invention relates to method for using clinical support algorithms and methods for training clinical support algorithms.

### BACKGROUND OF THE INVENTION

The performance of Clinical Decision Support Algorithms and Analytics and their specificity depends on the quality and consistency of the input data. However, the training data for developing and for applying these algorithms is currently captured from existing data streams that are intended for patient monitoring and real-time alarming.

Patient monitoring systems allow the caregiver to adjust the behavior of these existing data streams to optimize them for the specific environment, personal or hospital preference and the specific patient status. These adjustments (caregiver modifications) can significantly impact the data values and therefore the same (physiological) situation might be represented by a wide spread of clinically collected data values, which impacts the algorithm's development. The same uncertainty occurs again when such an algorithm is then used for a specific patient in a clinical context. It has been realized that avoiding this variability, which is not a physiological variability, would improve the performance of such algorithms.

The current approach for developing analytics is to collect data from a patient monitoring system by obtaining the data as it is provided in a data stream used for data interfaces. Data interfaces are used by caregivers to visualize physiological data on a corresponding user interface/display of the patient monitor or at the user interface of a connected system, such as a central station or an electrical medical record (EMR). This data stream is optimized by the caregiver for user interface purposes and for the alarming behavior of the patient monitoring system.

Some of the caregiver modifications can be mapped in a more or less unambiguous way to a standardized representation, e.g., the unit of measure. For analytics training purposes, this mapping can be done as part of the data collection process or at the time when the data is used for training.

However, conventionally, when the mapping takes place after the data was collected, the caregiver settings used to achieve the caregiver modifications are not stored with the data. Therefore, a correct and time specific mapping cannot always be achieved and thus the raw physiological data cannot be restored from the modified physiological data. When an analytics algorithm is used, such a mapping needs to take place such that the input data for the algorithm represents the format that the algorithm was trained with.

A major disadvantage of the current approach is that some caregiver modifications modify the data in such a way that the data cannot be mapped in an unambiguous way from one caregiver adjustments choice to another. Moreover, the mapping would require additional data that is not always available as part of the data collection process, e.g., ambient pressure. In these cases, the mappings are irreversible if there is not access to the necessary additional data.

This causes the data to deviate depending on the modifications that a caregiver has chosen, even for identical physiological conditions, thus providing modified data having "physiological uncertainty". As such, training a clinical support algorithm with the modified data can negatively impact the outputs of the algorithm during its application.

Similarly, the application of a clinical support algorithm using modified data causes the output to be further impacted by the physiological uncertainty due to possible further adjustments that a caregiver might apply to the data stream in order to control how the data is provided at the user interface. In particular, the modified data used in the application of the analytics algorithm may not be adapted in the same manner as the training data used on existing clinical support algorithms.

In summary, the current approach of using clinical support algorithms adds unpredictable variability to the outputs twice, once during training of the algorithm and once during application of the algorithm. This variability reduces the achievable precision of clinical support algorithm to predict a specific physiological condition.

One way to overcome these limitations would be to prevent the caregiver modifications and force the caregivers to abstain from optimizing the individual physiological data e.g., for displaying or for real-time alarming. However, caregivers often want to make modifications to the data stream to control how the data is provided at the user interface. Additionally, caregivers do not want to be limited in their freedom to modify the data stream for the purpose of training and/or using a clinical support algorithm. Such a solution is unlikely to be acceptable to caregivers and may negatively impact clinical care.

As such, there is a need for a solution which provides reduced variability in the output of clinical support algorithms without significantly impacting a caregiver's workflow or clinical care.

### SUMMARY OF THE INVENTION

The invention is defined by the independent claims. The dependent claims define advantageous embodiments.

According to examples in accordance with an aspect of the invention, there is provided a computer implemented method for collecting data indicative of the health of a subject, the method comprising:
obtaining raw physiological data of a subject;
inputting the raw physiological data into a clinical support algorithm trained on prior raw physiological data of one or more subjects to generate a first data stream containing the output of the clinical support algorithm; and
modifying the raw physiological data using caregiver settings to generate a second data stream containing the modified physiological data.

When using a trained algorithm, it is important that the representation of the inputs is consistent and standardized. However, patient monitoring systems process physiological data in a way to achieve a representation that is suited for use by caregivers via a user interface, which might eliminate important data content that might be beneficial for an algorithm like a clinical decision support algorithm. Furthermore, many caregivers want to control how the data is presented (e.g., at a user interface) by adapting/modifying the raw physiological data using caregiver settings. Many caregiver settings change the raw physiological to the extent that the modified physiological data can no longer be practically mapped to the representation preferred by the clinical support algorithm.

Caregiver settings are settings used to modify the raw physiological data for later use by the caregiver. For example, the modifications may be for the purpose of presenting/displaying the physiological data to caregivers and/or for the purpose of alarming. The caregiver settings may include settings set directly by the caregiver (i.e., caregiver-defined settings). In some cases, the caregiver settings may not be directly set by the caregiver. For example, some caregiver settings may be defined by safety and/or performance standards.

As such, it is proposed to generate two separate data streams using the raw physiological data. One of the data streams contains the output of the clinical decision algorithm, where the raw physiological data was used as an input for the clinical decision algorithm. The other data stream contains modified physiological data for use by the caregiver, where the modified physiological data is the raw physiological data modified according to the caregiver settings. For example, the raw physiological data may be modified according to the preferences of the caregiver (i.e., using caregiver-defined settings) and/or modified to achieve a particular representation of the data for ease of visualization by the caregiver (e.g., applying high-pass or low-pass filtering).

The output of the clinical support algorithm may then be displayed (e.g., to the caregiver). The output of the clinical support algorithm may also be modified according to the preferences of the caregiver (i.e., using caregiver-defined settings). Similarly, the modified physiological data (using the caregiver settings) may also be displayed to the caregiver. Of course, one or more of the outputs of the clinical support algorithm and the modified physiological data may also be stored for later use.

The preferred representation of the input data to the clinical support algorithm is based on the representation of the data which was used to train the clinical support algorithm. Said representation can be defined using data properties (e.g., unit of measurement, resolution, normalization method, correction method, offset, scale etc.) The representation/properties of the physiological data are often unknown from the physiological data per se unless explicitly specified in the physiological data.

Raw physiological data is data indicative of direct or indirect observations of variables attributable to the functioning of systems and/or subsystems in the subject's body. Raw physiological data is generally physiological data which has not been processed after being acquired. It must be understood that raw physiological data can still be the result of processing physical signals e.g., from sensors. Such processing of physical signal can involve several steps, like digitizing analog signals, demodulating light or filtering electrical sensor signals to the relevant physiological bandwidth.

A data stream, in general, contains data (in this case, raw physiological data) to convey information.

In the context of the invention, a clinical support algorithm is a trained algorithm that uses a subject's health information (i.e., the raw physiological data) to assist in making a diagnosis, treatment decisions, monitoring of the subject, deriving further physiological data or for similar purposes. For example, the clinical support algorithm may be a clinical decision support algorithm.

It will be appreciated that when the method is used with raw physiological data obtained from the same device (or similar devices), it can be assumed that the raw physiological data will have the same representation.

The method may comprise irreversibly modifying the raw physiological data using the caregiver settings to generate the second data stream.

Irreversibly modifying the raw physiological data means modifying the raw physiological data such that the raw physiological data cannot be derived from modified physiological data without additional information.

Modifying the raw physiological data may comprise processing the raw physiological data with one or more mathematical calculations to generate the modified physiological data.

Irreversibly modifying the raw physiological data using caregiver settings may comprise one or more of filtering the raw physiological data, averaging the raw physiological data over time, offsetting the raw physiological data, normalizing the raw physiological data, and calculating a subject-specific indexed representation of the raw physiological data. All of these adjustments irreversibly change the raw physiological data. Of course, other modifications will be known which irreversibly change the raw physiological data.

Inputting the raw physiological data into a clinical support algorithm may comprise adapting the raw physiological data using standardized processing prior to inputting the raw physiological data into the clinical support algorithm, wherein the prior raw physiological data on which the clinical support algorithm is trained is also adapted using the standardized processing.

In some cases, the representation of the raw physiological data may not be the preferred representation of the clinical support algorithm. In these cases, it is preferable to process (in the first data stream) the raw physiological data using standardized settings to adapt the representation of the raw physiological data to the preferred representation of the raw physiological data. In other words, the same standardized processing is used on the inputs to the clinical support algorithm as well as the data used to train the clinical support algorithm.

The standardized processing is typically different to the modifications due to the caregiver settings.

The standardized processing may be an initial processing/pre-processing of the raw physiological data. For example, the standardized processing may include removing outlier datapoints from the raw physiological data.

The raw physiological may comprise raw hemodynamic data of the subject. Often, hemodynamic data is adapted by the caregiver in such a manner that does not allow it to be mapped back to the raw hemodynamic data. For example, such data is often offset, normalized, corrected, filtered, averaged etc.

The method may further comprise storing and/or displaying the output of the clinical support algorithm and/or storing and/or displaying (512) the modified physiological data.

The first data stream and the second data stream may be output to the clinician.

The invention also provides a computer implemented method for training the clinical support algorithm with data indicative of the health of a subject, the method comprising:
obtaining raw physiological data of a subject; and
training a clinical support algorithm with the prior raw physiological data of the subject.

As with using clinical support algorithms, it is also important that the data on which they are trained have a consistent representation.

Training a clinical support algorithm with the prior raw physiological data may comprise adapting the raw physiological data applying standardized processing prior to training the clinical support algorithm with the raw physiological data.

The raw physiological may comprise raw hemodynamic data of the subject.

The invention also provides a computer program carrier comprising computer program code which, when executed on a processor, causes the processor to perform all of the steps of any of the aforementioned methods.

A computer program carrier may be, for example, a long-term storage product (e.g., non-volatile memory such a hard drive or a solid-state drive) or a bitstream.

The invention also provides a system for collecting data indicative of the health of a subject, the system comprising a processor configured to:
obtain raw physiological data of a subject;
input the raw physiological data into a clinical support algorithm trained on prior raw physiological data of one or more subjects to generate a first data stream containing the output of the clinical support algorithm; and
modify the raw physiological data using caregiver settings to generate a second data stream containing the modified physiological data.

The raw physiological may comprise raw hemodynamic data of the subject.

The processor may be configured to modify the raw physiological data based on caregiver settings by filtering the raw physiological data, averaging the raw physiological data over time, offsetting the raw physiological data, normalizing the raw physiological data and/or calculating an index using the raw physiological data.

The processor may be configured to irreversibly modify the raw physiological data using the caregiver settings to generate the second data stream.

The processor may be configured to input the raw physiological data into a clinical support algorithm by adapting the raw physiological data applying standardized processing prior to inputting the raw physiological data into the clinical support algorithm, wherein the prior raw physiological data on which the clinical support algorithm is trained is also adapted applying the standardized processing.

The system may further comprise one or more physiological measurement devices configured to obtain the raw physiological data.

The system may further comprise a display configured to display the output of the clinical support algorithm and/or configured to display the modified physiological data.

The system may also comprise a data storage device configured to store the output of the clinical support algorithm and/or configured to store the modified physiological data.

The invention also provides a computer implemented method for collecting data indicative of the health of a subject, the method comprising:
obtaining raw physiological data of a subject;
adapting the raw physiological data using standardized settings to generate standardized physiological data;
inputting the standardized physiological data into a clinical support algorithm trained on prior standardized physiological data of one or more subjects to generate a first data stream containing the output of the clinical support algorithm; and
modifying the raw physiological data using caregiver settings to generate a second data stream containing the modified physiological data.

All of the optional features described herein can also be used with the afore-mentioned method using standardized settings.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows a patient monitoring system inputting raw physiological data into a clinical support algorithm;
Fig. 2 shows the patient monitoring system inputting standardized physiological data into a clinical support algorithm;
Fig. 3 shows a patient monitoring system obtaining a training dataset;
Fig. 4 shows the patient monitoring system obtaining a standardized training dataset;
Fig. 5 shows a method for collecting data indicative of the health of a subject; and
Fig. 6 shows a system for collecting data indicative of the health of a subject.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a method for collecting data indicative of the health of a subject. The method comprises obtaining raw physiological data of a subject and inputting the raw physiological data into a clinical support algorithm, where the clinical support algorithm is trained on prior raw physiological data of one or more subjects. A first data stream containing the output of the clinical support algorithm is generated. The raw physiological data is also separately modified using caregiver settings to generate a second data stream containing the modified physiological data.

The invention can be used in an improved patient monitoring system to provide an additional independent data path, for the physiological data, that is not impacted by the caregiver modifications. Such caregiver modifications are performed to achieve a commonly accepted way to present physiological data to caregivers and/or modifications that an individual caregiver might want to make regarding how the physiological data is filtered, averaged, indexed, or presented based on a selected reference method (i.e., using caregiver settings to modify the physiological data). This improved patient monitoring system can be used for collecting the clinical data for algorithm development purposes (i.e., training) as well as when such an algorithm is used.

Thus, a caregiver can be provided with improved outputs from the clinical support algorithm (e.g., clinical user gets improved Clinical Decision Support Algorithms and Analytics) without constraining the clinical practice.

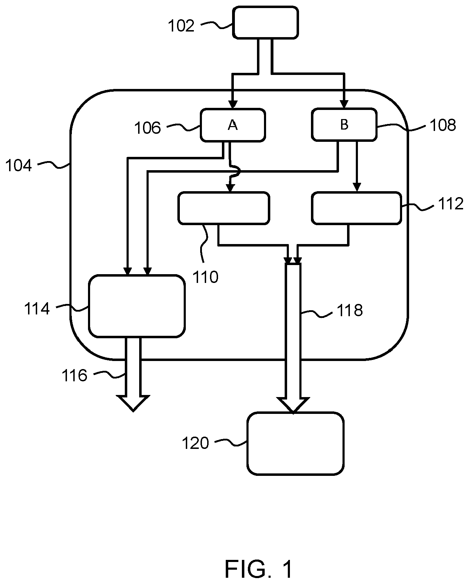

Fig. 1 shows a patient monitoring system 104 inputting raw physiological data 102 into a clinical support algorithm 114.

To overcome the above-described systematic deficiency of raw physiological data 102 with "physiological uncertainty" when using clinical support algorithms, a new approach is necessary. It is therefore proposed that, instead of using existing physiological data that is optimized for use by caregivers (e.g., for presenting the data or for alarming), additional data paths are provided directly between the raw physiological data 102 and the clinical support algorithm 114. As such, the input data for the clinical support algorithm 114 is not impacted by caregiver modifications.

In Fig. 1, the raw physiological data 102 comprises two separate physiological measurements, measurement 106 and measurement 108. In this case, there exists a data path directly between the measurements 106 and 108 and the clinical support algorithm 114. A first data stream 116, containing the output of the clinical support algorithm 114, is then generated. Of course, it will be appreciated that the raw physiological data 102 may comprise one or more measurements.

A patient monitoring system 104 may comprise at least one measurement function that is intended to provide physiological data to a caregiver. Such a patient monitoring system 104 may process the physiological data to achieve a commonly accepted way to present physiological data to caregivers and/or even allows the caregiver to adjust how the physiological data is processed to achieve a desired behavior when the physiological data is presented on the user interface or forwarded to another system components, e.g., for alarming or for remote viewing.

In Fig. 1, there also exists a separate data path between the measurements 106 and 108 and a display 120. The measurements 106 and 108 are modified using caregiver modifications 110 and 112 to achieve a commonly accepted way to present physiological data to caregivers and/or are modified based on caregiver setting (e.g., caregiver preferences). The caregiver modifications 110 and 112 can indicate caregiver settings to be used to modify the measurements 106 and 108. A second data stream 118 is then generated containing the modified measurements. The second data stream can then provide the modified measurements to a display (e.g., at a user interface) and/or may be otherwise used by the caregiver (e.g., for alarming or remote viewing purposes). More generally, the raw physiological data 102 is modified to achieve a target behavior for user interface purposes or it is modified based on caregiver settings. The modified physiological data is used by the caregiver.

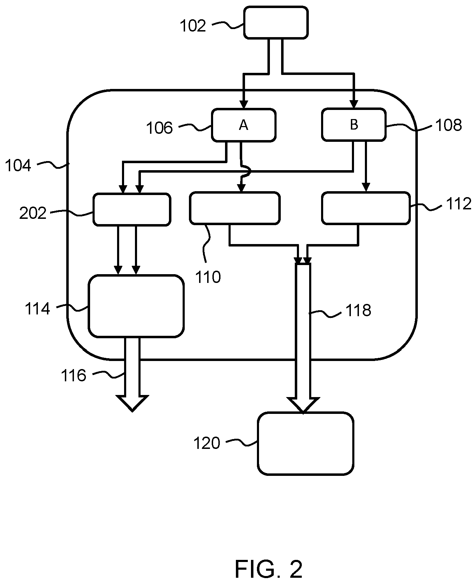

Fig. 2 shows the patient monitoring system 104 inputting standardized physiological data 202 into a clinical support algorithm 114. In this case, the raw physiological data 102 is adjusted based on standardized processing prior to being input into the clinical support algorithm 114. The standardized physiological data 202 is then input into the clinical support algorithm 114. The standardized processing may indicate standardized settings used for adjusting the raw physiological data 102.

In this case, the clinical support algorithm has also been trained with training data which has been adjusted based on the standardized processing.

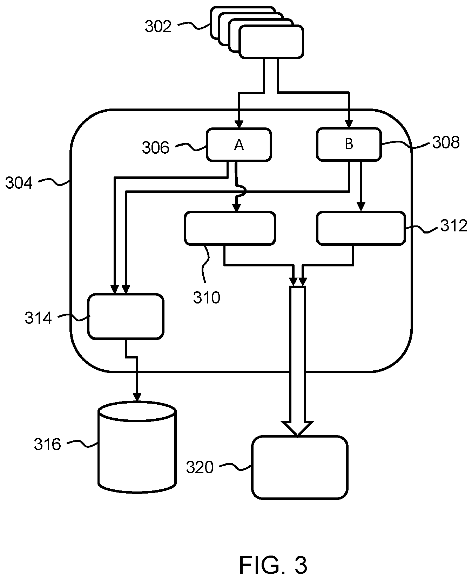

Fig. 3 shows a patient monitoring system 304 obtaining a training dataset 316. Raw physiological data 302 is obtaining from a plurality of subjects. In this case, the raw physiological data 302 comprises two separate physiological measurements from the plurality of subjects, measurements 306 and 308. A data path is provided directly between the measurements 306 and 308 and a data collection module 314. The raw physiological data 302 is then provided directly to the training dataset 316 without any caregiver modifications. A separate data path is provided between the measurements 306 and 308 and a display 320 or user interface. The caregiver modifications 310 and 312 can then be applied to the raw physiological data 302 without affecting the training data in the training dataset 316 being collected.

A clinical support algorithm can then be trained with the collected training data in the training dataset 316.

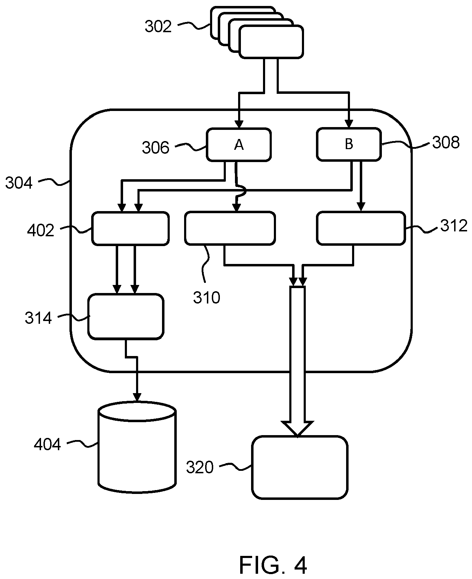

Fig. 4 shows the patient monitoring system 304 obtaining a standardized training dataset 404. As discussed for Fig. 2, the standardized training dataset 404 is used to train a clinical support algorithm when the clinical support algorithm is intended to be input with standardized physiological data. As such, the raw physiological data 302 is standardized and the standardized physiological data 402 is collected by the data collection module 314 and provided to the standardized training dataset 404 for use in training the clinical support algorithm.

Table 1 below shows examples of physiological data and corresponding settings that caregivers adjust which irreversibly impact the representation of the data (i.e., the physiological data is irreversibly modified) without knowledge of all of the inputs used to modify the physiological data. In other words, the caregiver settings shown in Table 1 cause modifications to raw physiological data which are irreversible per se and can only be reversed with knowledge of the inputs used to modify the raw physiological data. It is noted that said inputs are usually not stored, effectively making the modifications irreversible.

The caregiver settings may include settings set by the caregiver for the purpose of displaying the modified data at a user interface, setting alarms, or for remote viewing purposes. The caregiver settings may also, or alternatively, include settings set by an institution (e.g., a hospital) for the purpose of safety and/or performance standards.

**Table 1**

| **Data type** | **Adjustable data properties** | **Physiological Data** | **Caregiver Settings** |
|---|---|---|---|
| Wave samples | Normalization | Capnography | - Standard Temperature and Pressure |
| | | | - Barometric pressure compensated |
| | | | - Body Temperature Pressure Saturated (BTPS) |
| | | | - Ambient Temperature Pressure Dry (ATPD) |
| Numerical data | Normalization | - Cardiac output | - Indexed with body surface area (BSA) using Du Bois formula |
| | | - Stroke volume | |
| | | - Cardiac power output | - Indexed with BSA using Boyd formula |
| | | - Intrathoracic blood volume | |
| | | | - Indexed with BSA using Mosteller formula |
| | | - Global end-diastolic volume | |
| | | | - Indexed with Body Mass Index (BMI) |
| | | - Systemic vascular resistance | |
| | | | - Indexed with Body Fat Percentage (BFP), |
| | | - Pulmonary vascular resistance | |
| | | | - Indexed with Fat Free Mass Index (FFMI) |
| | | Extravascular Lung Water | - Indexed with Ideal Body Weight (IBW) |
| | | Capnography | - Standard Temperature and Pressure |
| | | | - Barometric pressure compensated |
| | | | - Body Temperature Pressure Saturated (BTPS) |
| | | | - Ambient Temperature Pressure Dry (ATPD) |

Adjusting raw physiological data based on the settings mentioned above can cause an irreversible change to the representation of the raw physiological data if additional information is not provided which would enable the reversal of said modifications. Currently, when obtaining training data for a clinical support algorithm, the additional information may not be available. Similarly, the additional information may not be available when using the modified physiological data as an input for a clinical support algorithm.

Table 2 below shows examples of physiological data and corresponding caregiver settings that caregivers adjust which irreversibly impact the raw physiological data. In these cases, the modified physiological data cannot be mapped back to a unified behavior even when additional information is provided.

**Table 2**

| **Data type** | **Adjustable data properties** | **Physiological Data** | **Caregiver settings** |
|---|---|---|---|
| Wave samples | Frequency filtering | Invasive pressure | - Diagnostic bandwidth (40 Hz) |
| | | | - Reduced bandwidth (12Hz) |
| | | ECG | - Diagnostic mode |
| | | | - Monitoring mode |
| | | | - Extended monitoring mode |
| | | Pleth waveform | - AC only |
| | Offset / Scale adjustment | Invasive Pressure wave | - Offset corrected |
| | | | - Auto scaled |
| | | | - Manually scaled |
| | Complex filtering | Pleth waveform | - Automatic scaling |
| | | | - Adjustable scaling |
| | | ECG | - AutoFilter to smooth the signal only during phases of disturbance |
| | | | - Fixed filtering |
| | | | - Pace pulse rejection On |
| | | | - Pace pulse rejection Off |
| | Artefact rejection | Any waveform | - Raw waveform |
| | | | - Smoothed waveform |
| | | Pleth waveform | - Fix delayed waveform |
| | | | - Variable delayed "scrubbed" waveform |
| Numerical data | Averaging | - SpO2 | Averaging time selection |
| | | - Pulse Rate | |
| | | - EEG numerics | |
| | | - Blood pressure numerics | |
| | Type of measurement algorithm | Noninvasive Blood pressure | - Measurement reference method "Auscultatory" (manual cuff) |
| | | | - Measurement reference method "Invasive" (intra-arterial) radial artery. |
| | | | - Measurement reference method "Invasive" (intra-arterial) femoral artery. |
| | | Heart rate | - Averaged |
| | | | - Beat-to-beat |
| | | Pulse rate or SpO2 from pulse oximetry | - Averaged |
| | | | - Beat-to-beat |
| | Artefact rejection | Blood pressure | Artifact Suppression on/off |
| | | Pulse rate or SpO2 from pulse oximetry | - High Sensitivity |
| | | | - Normal sensitivity |
| | Resolution | Temperature | - 0.5 °C |
| | | | - 0.1 °C |
| | | | - 0.05 °C |
| | | | - 0.01 °C |
| | | | - 0.001 °C |

Using any of the caregiver settings defined in Tables 1 and 2 above can often aid the caregiver when interpreting the physiological data, especially when compared with interpreting raw physiological data. However, clinical support algorithms may work best with raw physiological data or specific standardized representations of the input data. As such, it is proposed to use the raw physiological data as an input to the clinical support algorithm whilst providing the raw physiological data for modification based on the user interface needs and caregiver settings separately. The raw physiological data can be adapted based on standardized processing prior to being input into the clinical support algorithm, where the clinical support algorithm is trained with raw physiological data adapted with the same standardized processing. The standardized processing may be different to the caregiver settings.

In a first example, the standardized processing for non-invasive blood pressure (NiBP) measurements may define that the NiBP values are calibrated for invasive reference independently from the customer configuration (which could be either invasive reference or auscultatory reference).

In a second example, the standardized processing for hemodynamic measurements may define the use of a standardized BSA method to index the hemodynamic parameters.

In a third example, the standardized processing for the raw physiological data may define a standardized unit for the measurement independent for the selected unit for presentation (e.g., kPa, mmHg, cmO2 etc.).

The approach described herein can also be applied in distributed patient monitoring systems where the measurement function, the data storage, the modifications to the raw physiological data, the use of the data as well as the provision of the output of the clinical support algorithm can be distributed across different devices. For example, a telemetry transmitter and a central station may form part of the patient monitoring system. Additionally, applications of the approach in context of electronic medical records or cloud-based analytics can also benefit from the approach described herein.

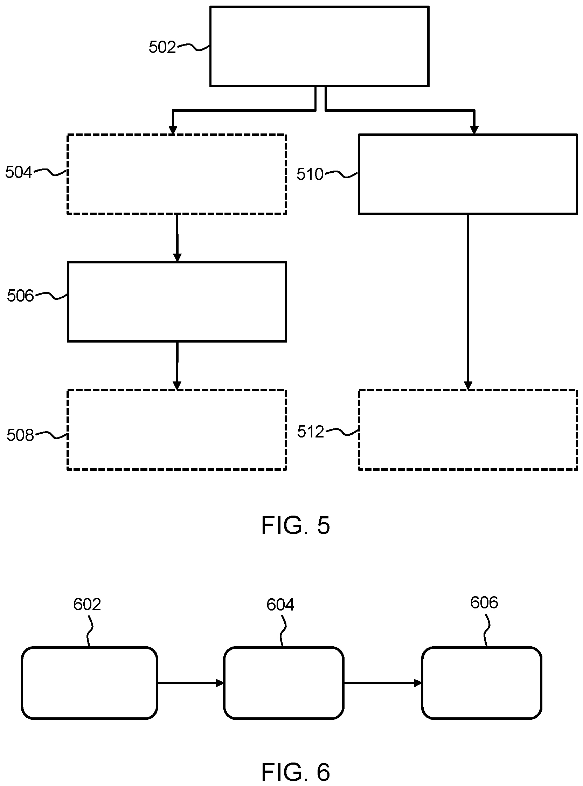

Fig. 5 shows a method for collecting data indicative of the health of a subject. In step 502, raw physiological data of a subject is obtained. For example, the raw physiological data may be measured using a physiological measurement device. In step 506, the raw physiological data is input into a clinical support algorithm. The clinical support algorithm has been trained on prior raw physiological data of one or more subjects. A first data stream containing the output of the clinical support algorithm is thus generated. Optionally in step 508, the output of the clinical support algorithm is displayed and/or stored.

In step 504, the raw physiological data may be adapted using standardized processing prior to being input into the clinical support algorithm.

In step 510, the raw physiological data is modified using caregiver settings to generate a second data stream containing the modified physiological data. Optionally, the modified physiological data can be displayed and/or stored in step 512.

The standardized processing in general is different from the possible modifications to the raw physiological based on the caregiver settings. But it can also occur that a specific combination of caregiver settings results in the same behavior as the standardized processing.

Fig. 6 shows a system for collecting data indicative of the health of a subject. The system comprises one or more physiological measurement devices 602 configured to obtain raw physiological data, a processor 604 capable of performing all of the steps shown in Fig. 5 and a display 606 for displaying the output of the clinical support algorithm and/or the modified physiological data. The system may also comprise a non-volatile memory for storing the output of the clinical support algorithm and/or the modified physiological data.

The skilled person would be readily capable of developing a processor for carrying out any herein described method. Thus, each step of a flow chart may represent a different action performed by a processor and may be performed by a respective module of the processing processor.

As discussed above, the system makes use of a processor to perform the data processing. The processor can be implemented in numerous ways, with software and/or hardware, to perform the various functions required. The processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A computer implemented method for collecting data indicative of the health of a subject, the method comprising:
obtaining (502) raw physiological data of a subject;
inputting (506) the raw physiological data into a clinical support algorithm trained on prior raw physiological data of one or more subjects to generate a first data stream containing the output of the clinical support algorithm; and
modifying (510) the raw physiological data using caregiver settings to generate a second data stream containing the modified physiological data.

2. The method of claim 1, comprising irreversibly modifying the raw physiological data using the caregiver settings to generate the second data stream.

3. The method of claim 2, wherein irreversibly modifying the raw physiological data using caregiver settings comprises one or more of:
filtering the raw physiological data;
averaging the raw physiological data over time;
offsetting the raw physiological data;
normalizing the raw physiological data; and
calculating a subject-specific indexed representation of the raw physiological data.

4. The method of any of claims 1 to 3, wherein inputting the raw physiological data into the clinical support algorithm comprises adapting (504) the raw physiological data using standardized processing prior to inputting the raw physiological data into the clinical support algorithm, wherein the prior raw physiological data on which the clinical support algorithm is trained is also adapted using the standardized processing.

5. The method of any of claims 1 to 4, wherein the raw physiological data comprises raw hemodynamic data of the subject.

6. The method of any of claims 1 to 5, further comprising:
storing and/or displaying (508) the output of the clinical support algorithm; and/or
storing and/or displaying (512) the modified physiological data.

7. A computer implemented method for training the clinical support algorithm of any of claims 1 to 5 with data indicative of the health of a subject, the method comprising:
obtaining raw physiological data of a subject; and
training a clinical support algorithm with the prior raw physiological data of the subject.

8. The method of claim 7, wherein training a clinical support algorithm with the prior raw physiological data comprises adapting the raw physiological data applying standardized processing prior to training the clinical support algorithm with the raw physiological data.

9. The method of claims 7 or 8, wherein the raw physiological data comprises raw hemodynamic data of the subject.

10. A computer program carrier comprising computer program code which, when executed on a processor, causes the processor to perform all of the steps of the method according to any of the preceding claims.

11. A system for collecting data indicative of the health of a subject, the system comprising a processor (604) configured to:
obtain raw physiological data (102) of a subject;
input the raw physiological data into a clinical support algorithm (114) trained on prior raw physiological data of one or more subjects to generate a first data stream (116) containing the output of the clinical support algorithm; and
modify the raw physiological data using caregiver settings to generate a second data stream (118) containing the modified physiological data.

12. The system of claim 11, wherein the processor is configured to irreversibly modify the raw physiological data using the caregiver settings to generate the second data stream.

13. The system of claims 11 or 12, wherein the processor is configured to input the raw physiological data into a clinical support algorithm by adapting the raw physiological data applying standardized processing prior to inputting the raw physiological data into the clinical support algorithm, wherein the prior raw physiological data on which the clinical support algorithm is trained is also adapted applying the standardized processing.

14. The system of any of claims 11 to 13, further comprising one or more physiological measurement devices (602) configured to obtain the raw physiological data.

15. The system of claims 13 or 14, further comprising a display (606) configured to display the output of the clinical support algorithm and/or configured to display the modified physiological data.
